(19) Europäisches Patentamt
European Patent Office
Office européen des brevets

(11) **EP 2 153 857 B1**

(12) **EUROPÄISCHE PATENTSCHRIFT**

(45) Veröffentlichungstag und Bekanntmachung des Hinweises auf die Patenterteilung:
**17.01.2018 Patentblatt 2018/03**

(51) Int Cl.:
***A61M 16/00*** *(2006.01)*

(21) Anmeldenummer: **09400038.7**

(22) Anmeldetag: **10.08.2009**

(54) **Vorrichtung zur Beatmung**

Device for artificial ventilation

Dispositif d'assistance respiratoire

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **11.08.2008 DE 102008038267**

(43) Veröffentlichungstag der Anmeldung:
**17.02.2010 Patentblatt 2010/07**

(73) Patentinhaber: **Löwenstein Medical Technology S.A.**
**2557 Luxembourg (LU)**

(72) Erfinder:
• **Tiemann, Björn**
**22926 Ahrensburg (DE)**

• **Göbel, Christoph, Dr.**
**22457 Hamburg (DE)**
• **Adametz, Benjamin**
**22767 Hamburg (DE)**

(74) Vertreter: **Patentanwälte Klickow & Partner Partnerschaftsgesellschaft mbB**
**Jessenstraße 4**
**22767 Hamburg (DE)**

(56) Entgegenhaltungen:
**EP-A1- 0 521 314     EP-A1- 1 346 743**
**EP-A1- 1 586 343     WO-A1-02/28460**

**Beschreibung**

Die Erfindung betrifft eine Vorrichtung zur Beatmung, die eine mit einer Steuerung verbundene Atemgaspumpe aufweist und bei der die Atemgaspumpe mit einem Patienteninterface verbindbar ist sowie bei der von der Steuerung während einer Exspirationsphase ein niedrigerer Atemgasdruck als während einer Inspirationsphase vorgegeben ist und bei der zur messtechnischen Erfassung mindestens eines Parameters des Atemgases mindestens ein Sensor an die Steuerung angeschlossen ist wobei die Steuerung eine Steuercharakteristik mit einer Mindestexspirationszeit aufweist.

Insbesondere betrifft die Erfindung eine entsprechende Vorrichtung, bei der ein Beginn der Inspirationsphase von einem in Abhängigkeit vom messtechnisch erfassten Parameter generierten Triggersignal gesteuert ist.

Mit derartigen Vorrichtungen ist es alternativ oder kumulativ möglich, eine Triggerung durch den Patienten über Druck oder Fluss, durchzuführen.

Derartige Vorrichtungen können insbesondere im Zusammenhang mit Beatmungsgeräten zum Einsatz kommen, die für Patienten mit COPD-Erkrankungen verwendet werden. Bei derartigen Erkrankungen sind die Lungen der Patienten in der Regel stark geschädigt. Typische Krankheitsbilder sind Emphysem und chronisch obstruktive Bronchitis. Diese Erkrankungen führen zu Rückkopplungen auf messtechnisch erfassbare Signale und beeinflussen insbesondere den Volumenfluß des Atemgases. Die Störungen machen sich in unregelmäßigen Signalverläufen bei einer messtechnischen Erfassung von Atemgasparametern bemerkbar.

Bei Beatmungsgeräten, die in Abhängigkeit von messtechnisch erfassten Signalen gesteuert werden, führen diese Störungen zu Fehlfunktionen, die therapeutisch sinnvolle Inspirationszeiten und Exspirationszeiten verändern und insbesondere zu einer ungewollten Verkürzung von Exspirationszeiten führen.-

Aus der EP 1 586 343 A1 ist bereits ein Verfahren und eine Vorrichtung zur kontrollierten oder mandatorischen Beatmung bekannt. Die Atemarbeit des Patienten wird hierbei ständig vom Beatmungsgerät übernommen. Das Beatmungsgerät gibt die zeitliche Dauer der Inspirationsphasen und der Exspirationsphasen vor.

In der WO 02/28460 A1 sowie der EP 0 521 314 A1 werden bereits Verfahren zur Durchführung einer Beatmung in Abhängigkeit von Triggersignalen beschrieben. Insbesondere erfolgt eine Umschaltung von der Inspirationsphase in die Exspirationsphase in Abhängigkeit von Triggersignalen, die durch eine sensorische Erfassung von eigenen Atembemühungen des Patienten bereitgestellt werden.

Aufgabe der vorliegenden Erfindung ist es, eine Vorrichtung zur unterstützenden Beatmung, der einleitend genannten Art derart zu konstruieren, dass eine fehlertolerante Gerätesteuerung unterstützt wird. Diese Vorrichtung ist in Anspruch 1 definiert. Insbesondere ist bei dieser Vorrichtung, bei der Beginn der Inspirationsphase von einem in Abhängigkeit vom messtechnisch erfassten Parameter generierten Triggersignal gesteuert wird, daran gedacht, dass die Steuerung mit einer Triggersignalunterdrückung versehen ist, die eine Druckerhöhung für eine vorgebbare Blockadezeit sperrt, die mit einer exspiratorischen Druckabsenkung beginnt.

Erfindungsgemäß wird berücksichtigt, dass zu einem Beginn der Exspiration, die mit einem vergleichsweise großen Volumenstrom an Atemgas im Zusammenhang steht, starke Störungen des Flowsignales vorliegen. Dieses Signal wird typischerweise Messtechnisch erfasst, um bei einer druckgesteuerten Beatmung nach einer entsprechenden gerätetechnischen Signalauswertung zur Generierung eines Triggersignals verwendet zu werden. Zum Ende der Exspirationszeit liegt ein vergleichsweise kleiner Volumenstrom vor und das Signal ist deutlich weniger gestört. Bei Patienten mit COPD-Erkrankungen tritt das zusätzliche Problem auf, das im Vergleich zu anderen Erkrankungen der Lunge zu einem Exspirationsbeginn lediglich ein geringer Volumenstrom detektierbar ist, der eine aufwendige Triggersignalerkennung im Beatmungsgerät erfordert.

Durch die erfindungsgemäße Triggersignalunterdrückung während der Zeit der höchsten Störwahrscheinlichkeit wird eine ungewollte Verkürzung der Exspiration vermieden. Die Blockadezeit vermeidet die Verwendung unempfindlicher Signalauswertungen, da die stark gestörten Signale der Auswertung nicht zugeführt werden und im Anschluss an das Auftreten der stark gestörten Signale eine empfindliche Signalauswertung realisierbar ist.

Eine vorteilhafte Gerätesteuerung kann dadurch erfolgen, dass das Triggersignal durch Auswertung eines Signales eines Volumenstromes an Atemgas ermittelt wird.

Eine Erkennung eines Endes der Exspirationsphase kann dadurch erfolgen, dass ein Vorzeichenwechsel einer ersten zeitlichen Ableitung des Flowsignals zur Triggerauslösung verwendet wird.

Alternativ ist auch daran gedacht, dass ein Vorzeichenwechsel einer zweiten zeitlichen Ableitung des Flowsignals zur Triggerauslösung verwendet wird.

Gemäß einer vereinfachten Ausführungsvariante ist es möglich, dass die Blockadezeit fest vorgegeben ist.

Ein verbesserter Gerätekomfort wird dadurch bereitgestellt, dass die Blockadezeit adaptiv von einer Messsignalauswertung ermittelt wird.

In den Zeichnungen sind Ausführungsbeispiele der Erfindung schematisch dargestellt. Es zeigen:

Fig. 1:    eine schematische Darstellung eines Beatmungsgerätes mit angeschlossenem Patienteninterface,

Fig. 2:    einen Verlauf eines Volumenstromes an Atemgas ohne Störungen,

Fig. 3: einen gestörten Verlauf des Volumenstromes an Atemgas bei COPD-Erkrankungen und

Fig. 4: eine weitere Darstellung eines Volumenstromes mit verlängerter Exspirationszeit.

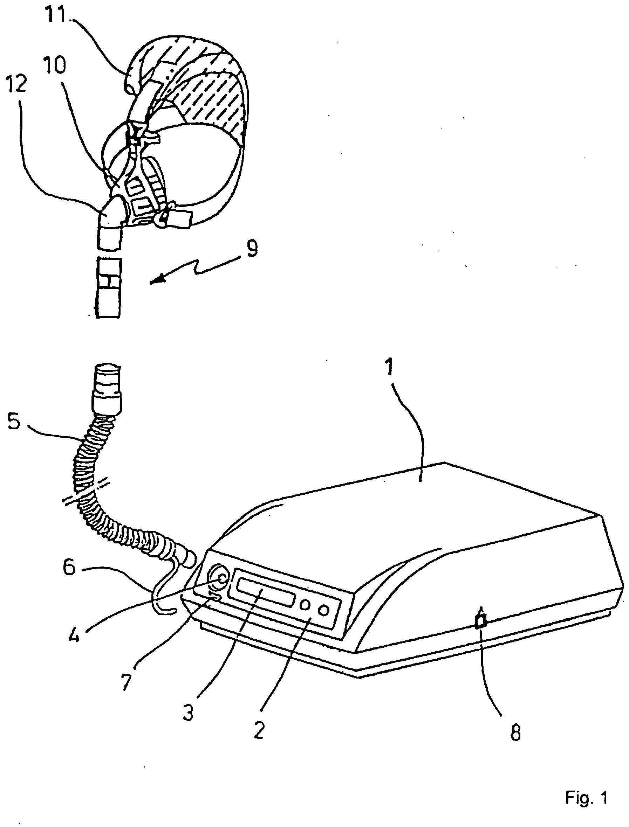

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Atemgasschlauch (5) angeschlossen. Entlang des Atemgasschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

An der dem Gerätegehäuse (1) abgewandten Seite des Beatmungsschlauches (5) befindet sich eine Schlauchankopplung (9) zur Verbindung des Beatmungsschlauches (5) mit der Atemmaske (10).

Fig. 1 zeigt darüber hinaus eine Atemmaske (10), die als Nasenmaske oder als Vollgesichtsmaske ausgebildet sein kann. Eine Fixierung im Bereich des Kopfes eines Patienten kann über eine Kopfhaube (11) erfolgen.

Die Atemmaske (10) weist als Schlauchanschluss (13) einen gebogenen Kugelgelenkanschluss (12) auf, der über die Schlauchankopplung (9) mit dem Beatmungsschlauch (5) verbunden wird.

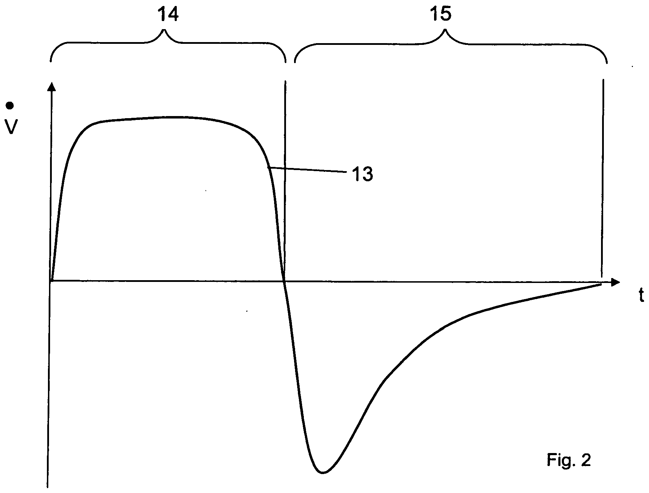

Fig. 2 zeigt einen Verlauf eines Volumentstromes (13), der in eine Inspirationsphase (14) und eine Exspirationsphase (15) unterteilt ist. Während der Inspirationsphase (14) stellt das Beatmungsgerät einen erhöhten und während der Exspirationsphase (15) einen verminderten Druck bereit.

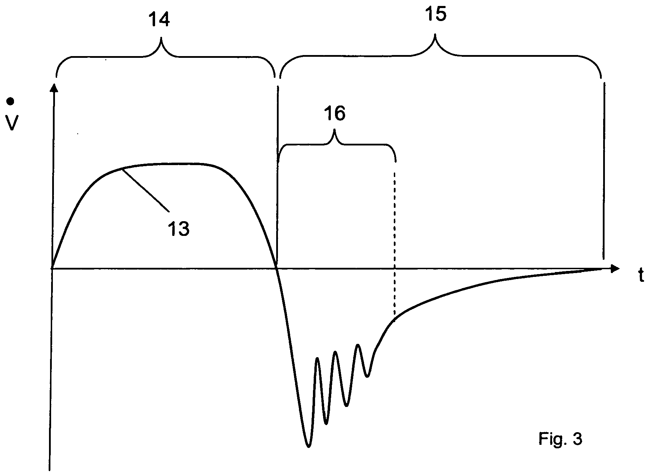

Fig. 3 zeigt einen typischen Verlauf des Volumenstromes (13) für Patienten mit COPD-Erkrankungen. Der Volumenstrom (13) in der Inspirationsphase (14) ist stark abgeflacht und während der Exspirationsphase (15) treten insbesondere zu einem Beginn dieser Phase erhebliche Störungen auf. Die Störungen führen zu starken Schwankungen des Volumenstromes sowie gegebenenfalls sogar zu einem Rückflow.

Eingezeichnet in Fig. 3 ist eine Blockadezeit (16), während der eine Erfassung eines Messsignales zur Generierung eines Triggersignales und/oder eine Auswertung des Messsignales zur Bereitstellung des Triggersignales und/oder eine Ausgabe des Triggersignales und/oder eine Funktionsauslösung aufgrund eines Triggersignales nicht durchgeführt wird. Eine Fehlfunktion des Gerätes aufgrund einer fälschlich detektierten Beendigung der Exspirationsphase (15) wird somit vermieden und hierdurch für eine ausreichend lange Exspirationsphase (15) gesorgt. Das Messsignal kann direkt oder nach einer Filterung ausgewertet werden.

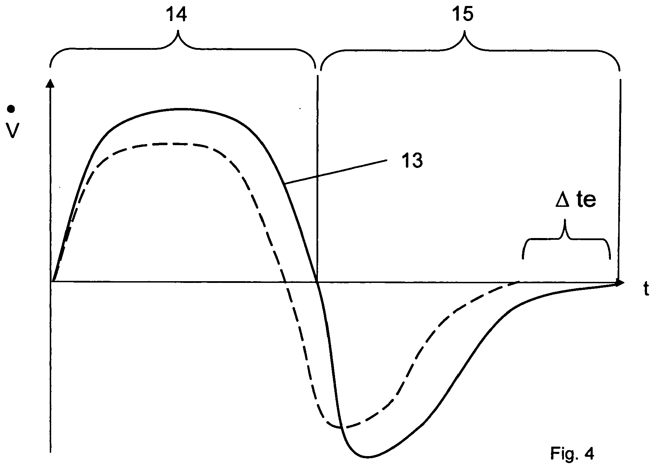

Fig. 4 zeigt einen weiteren Verlauf des Volumenstromes (13) an, bei dem die Exspirationsphase (15) verlängert wurde.

Hinsichtlich der zeitlichen Vorgabe der Blockadezeit (16) sind unterschiedliche Varianten möglich. Zum einen kann die Blockadezeit (16) fest eingestellt sein. Ebenfalls ist es möglich, die Blockadezeit (16) adaptiv vom Beatmungsgerät selbst ermitteln zu lassen. Die Ermittlung kann aufgrund einer Analyse der Messwerte erfolgen, bei der die Zeiträume großer Störungen erfasst werden. Alternativ oder ergänzend zur vorstehend erläuterten Triggerblockade ist es auch möglich, die Exspirationsphase zeitlich gesteuert zu verlängern. Gemäß einem Ausführungsbeispiel ist daran gedacht, jede fünfte Exspirationsphase gegenüber einer Grundlänge um etwa 20% zu verlängern. Ebenfalls ist es alternativ oder ergänzend möglich, die Exspirationszeit in Abhängigkeit vom inspirierten Volumen an Atemgas zu verlängern. Ein größeres Volumen an Atemgas während der Inspirationszeit führt somit zu einer entsprechenden Verlängerung der Exspirationszeit.

Diese Art der Gerätesteuerung kann bei einer assistierten Beatmung mit Hintergrundfrequenz (Beispiel: ST-Modus) für die adaptive Steuerung der Hintergrundfrequenz zur Anwendung kommen. Alternativ ist eine kontrollierte Beatmung (z.B. T-Modus) möglich, indem eine fest vorgegebene durch diese adaptive Exspirationszeit ersetzt wird.

Eine adaptive Ermittlung der Exspirationsphase (15) kann unter Berücksichtigung der nachfolgenden Berechnungsmethoden erfolgen, die im Steuerprogramm des Beatmungsgerätes implementierbar sind.

Für die zumeist passive Exspiration eines Patienten gilt die Bewegungsgleichung (Equation of Motion) der Beatmung:

$$Paw = R \cdot flow + \frac{1}{C} \cdot \int flow$$

wobei Paw der durch das Beatmungsgerät aufgebrachte exspiratorische Druck ist. R ist der Atemwegswiderstand und C die Compliance (Dehnbarkeit) der Lunge.

Nach Lösung dieser Differentialgleichung ergibt sich für den zeitlichen Verlauf des exspiratorischen Volumens:

$$V(t) = VT \cdot e^{\frac{-t}{\tau}}$$

Wobei $\tau = R \cdot C$ gilt.

Wenn beim Patienten allerhöchstens z.B. Vsoll = 10ml am Ende der Exspiration in seiner Lunge verbleiben dürfen, dann ergibt sich für Texsp:

$$Texsp = -\tau \cdot \ln\left(\frac{v_{soll}}{v_T}\right)$$

Die Zeitkonstante $\tau$ kann z.B. aus einer Lungenfunktionsmessung bekannt sein, oder z.B. in der Analysephase eines TA-Modus bestimmt worden sein.

Das Volumen VT kann entweder online pro Atemzug gemessen werden oder sich als Durchschnittsbetrachtung aus mehreren Atemzügen ergeben.

Gemäß einer weiteren Ausführungsform ist es möglich, die Steuerung der Exspirationszeit bei der assistierten Beatmung mit Hintergrundfrequenz oder bei der kontrollierten Beatmung direkt auf Basis des Flusssignals während der Exspiration zu steuern. Die Gerätesteuerung erlaubt erst dann ein Umschalten in die nachfolgende Inspiration, wenn keine Ausatemaktivität des Patienten mehr erkannt wird, der Exspirationsfluss - nach Abzug eines ggf. vorhandenen Leckageflusses - zu Null geworden ist.

Bei der gerätetechnischen Realisierung kann die Generierung des Triggersignales für den Inspirationsbeginn beispielsweise derart erfolgen, dass die erste oder zweite zeitliche Ableitung des Volumenstromes (13) erfasst wird. Insbesondere ist hierbei an eine gefilterte Signalauswertung gedacht.

Bei einer unterstützten Beatmung erfolgt bei detektierten Fehlmessungen eine Unterdrückung des Triggersignales. Bei der assistierten Beatmung erfolgt eine Freigabe für die Auswertung der Steigung des Flowsignals nach Ablauf der Mindestexspirationszeit. Typischerweise wird bei einer erkannten Steigung des Flowsignals von 0 in die Inspiration umgeschaltet.

## Patentansprüche

1. Vorrichtung zur unterstützenden Beatmung, die eine mit einer Steuerung verbundene Atemgaspumpe aufweist und bei der die Atemgaspumpe mit einem Patienteninterface verbindbar ist, sowie bei der von der Steuerung während einer Exspirationsphase ein niedrigerer Atemgasdruck als während einer Inspirationsphase vorgegeben ist und bei der zur messtechnischen Erfassung mindestens eines Parameters des Atemgases mindestens ein Sensor an die Steuerung angeschlossen ist, **dadurch gekennzeichnet, dass** die Steuerung eine Steuercharakteristik mit einer Mindestexspirationszeit aufweist, die eine Triggerblockadezeit ist, während der ein registriertes patientenseitiges Signal, auf dessen Basis eine Umschaltung in die Inspiration möglich ist, nicht berücksichtigt wird und dass die Steuerung mit einer Triggersignalunterdrückung versehen ist, die eine Druckerhöhung für die vorgebbare Triggerblockadezeit sperrt, die mit einer exspiratorischen Druckabsenkung beginnt, und dass die die Mindestexspirationszeit um die Zeitdauer bis zum Auftreten eines den Atemfluss der Exspiration oder das Atemzugvolumen der Inspiration repräsentierenden Signals, das anzeigt, dass der Exspirationsvorgang des Patienten abgeschlossen ist, verlängert wird, falls nach Ablauf der Mindestexspirationszeit angezeigt wird, dass der Exspirationsvorgang nicht abgeschlossen ist.

2. Vorrichtung nach Anspruch 1, **dadurch gekennzeichnet, dass** als Atemgas-Parameter der Fluss oder der Druck oder das Volumen an Atemgas erfasst wird und Steuervorgänge auf Basis des sensorisch erfassten Parameters erfolgen.

3. Vorrichtung nach Anspruch 1 oder 2, **dadurch gekennzeichnet, dass** die Steuerung den Atemgasdruck in der Exspiration erhöht, wenn ein den Atemfluss oder das Atemzugvolumen repräsentierendes Signal anzeigt, dass der Exspirationsvorgang des Patienten nicht beendet ist.

## Claims

1. Apparatus for supportive ventilation, which has a respiratory gas pump connected to a controller and in which the respiratory gas pump can be connected to a patient interface and in which the controller predefines a lower respiratory gas pressure during an expiration phase than during an inspiration phase, and in which at least one sensor is connected to the controller for the purpose of capturing at least one parameter of the respiratory gas using metrology, **characterized in that** the controller has a control characteristic with a minimum expiration time which is a trigger blockade time during which a registered patient signal, on the basis of which it is possible to change over to inspiration, is not taken into account, and **in that** the controller is provided with a trigger signal suppression means which blocks a pressure increase for the predefinable trigger blockade time which begins with an expiratory pressure reduction, and **in that** the minimum expiration time is extended by the period until the occurrence of a signal, which represents the respiratory flow of the expiration or the breathing volume of the inspiration and indicates that the expiration process of the patient has been concluded, if, after expiry of the minimum expiration time, it is indicated that the expiration process has not been concluded.

2. Apparatus according to Claim 1, **characterized in that** the flow or the pressure or the volume of respiratory gas is captured as the respiratory gas parameter and control operations are carried out on the basis of the parameter captured using sensors.

3. Apparatus according to Claim 1 or 2, **characterized in that** the controller increases the respiratory gas pressure in the expiration if a signal representing the respiratory flow or the breathing volume indicates that the expiration process of the patient has not finished.

**Revendications**

1. Dispositif d'assistance respiratoire, comportant une pompe à gaz respiratoire connectée à un contrôleur et où la pompe à gaz respiratoire peut être connectée à une interface patient, où le contrôleur impose lors d'une phase d'expiration une pression de gaz respiratoire plus faible que lors d'une phase d'inspiration et où au moins un capteur est raccordé au contrôleur afin de mesurer au moins un des paramètres du gaz respiratoire,
**caractérisé en ce que** le contrôleur comprend au moins une caractéristique de contrôle avec une durée minimale d'expiration qui soit une durée de blocage de déclenchement, pendant laquelle un signal enregistré du côté patient, sur la base duquel une commutation vers l'inspiration est possible, ne sera pas pris en compte et
**en ce que** le contrôleur est pourvu d'une inhibition de signal déclencheur, qui verrouille une élévation de pression pendant la durée de blocage de déclenchement spécifiée, et
**en ce que** le temps minimal d'expiration est prolongé jusqu'à l'apparition d'un signal représentant le débit d'expiration ou le volume d'inspiration qui indique que le processus d'expiration du patient est terminé, au cas où le processus d'expiration ne soit pas terminé après écoulement du temps minimal d'expiration.

2. Dispositif selon la revendication 1, **caractérisé en ce qu'**est mesuré le débit, la pression ou le volume de gaz respiratoire et que des processus de contrôle s'ensuivent sur la base du paramètre mesuré.

3. Dispositif selon la revendication 1 ou 2, **caractérisé en ce que** le contrôle de la pression de gaz respiratoire augmente si un signal représentatif du débit respiratoire ou du volume respiratoire indique que le processus d'expiration du patient n'est pas terminé.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1586343 A1
- WO 0228460 A1

- EP 0521314 A1